# EUROPEAN PATENT APPLICATION

(11) **EP 1 674 103 A1**
(43) Date of publication of application: **28.06.2006**
(21) Application number: 04106998.0
(22) Date of filing: 27.12.2004
(51) Int. Cl.: A61K 31/4985, C07D 487/04, A61P 25/00

(54) **Arylpiperazine derivatives and use thereof as 5-HT1A receptor ligands**

(71) Applicant: Cepa Schwarz Pharma s.l., 28046 Madrid (ES)
(72) Inventor: Lopez Rodriguez, Maria Luz, 28040, Madrid (ES); Benhamú Salama, Bellinda, 28100, Alcobendas (Madrid) (ES); Valhondo Falcón, Margarita, 28024, Madrid (ES)
(74) Representative: Barlocci, Anna

(57) **Abstract**

Novel substituted arylpiperazine derivatives with activity as 5-hydroxytryptamine 1A (5-HT_{1A}) receptor subtype ligands, to their stereochemical isomers, methods of their preparation, and to their use and to pharmaceutical compositions containing them for the treatment of Parkinson disease, cerebral damage by thromboembolic ictus, craneoencephalic traumatisms, depression, migraine, pain, psychosis, anxiety disorders, aggressive disorders or urinary tract disorders.

## Description

### FIELD OF THE INVENTION

The present invention relates to arylpiperazine derivatives and, in particular, to their activity as 5-hydroxytryptamine 1A (5-HT_{1A}) receptor subtype ligands, to their stereochemical isomers and to their use and to pharmaceutical compositions containing them for the treatment of pathological states for which a ligand of these receptors is indicated.

### BACKGROUND ART

5-HT_{1A} receptor is a major target for neurobiological research and drug development due to its implication in many (patho)physiological processes. 5-HT_{1A} ligands have been proven to be effective in anxiety and depression. In addition to therapeutic applications in the field of psychiatry, more recent preclinical studies have suggested that 5-HT_{1A} receptor ligands have also pronounced neuroprotective properties.

5-HT_{1A} ligands may find use in the treatment of several diseases such as anxiety, depression, schizophrenia, sexual dysfunction, cognitive deficits resulting from neurodegenerative diseases like Alzheimer's Disease, nausea and vomiting, sleep disorders, pain, obesity, pain, addiction/withdrawal and in the treatment of prostate cancer. More recent evidence now indicates that 5-HT_{1A} ligands act in other disease states and conditions by virtue of their ability to inhibit the release of glutamate. 5-HT_{1A} ligands may be used to treat conditions arising from the dysfunction of the glutamate neurotransmitter system or the aberrant release of glutamate.

Glutamate is the predominant neurotransmitter in the central nervous system and it plays an important role in neuroplasticity. As such, excessive extracellular levels of glutamate have been associated with the pathophysiology of both acute neurodegenerative disorders such as stroke, transient ischemic attack and spinal/brain trauma, as well as chronic neurodegenerative disorders such as epilepsy, Alzheimer's Disease, amyotrophic lateral sclerosis, Huntington's Disease, Parkinson's Disease, AIDS dementia and retinal diseases. Compounds which inhibit or attenuate the release of glutamate represent potential neuroprotective agents for the treatment of ischemia resulting from stroke, transient ischemic attack, brain/spinal trauma and fetal hypoxia (Koroshetz, W. J. and Moskowitz, M. A., Emerging Treatment for Stroke in Humans. Trends in Pharmacol. Sci. 1996, 17, 227-233).

WO 96/06846 relates to arylpiperazine derivatives of formula I 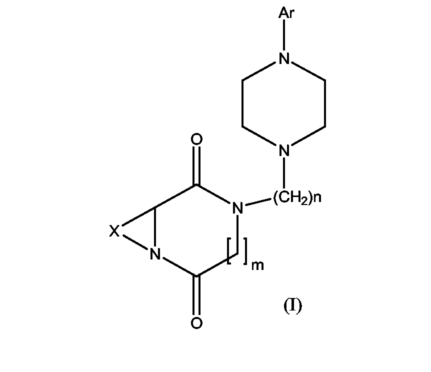 wherein X is -(CH₂)₃- or -(CH₂)₄)-; m = 0 or 1; n = 1 to 4; Ar = 1-naphthyl, 7-benzofuranyl, 2,3-dihydro-1,4-benzodioxan-5-yl, 3,4-dihydro-2*H*-1,5-benzodioxepin-6-yl, phenyl or phenyl substituted by alkyl, halogen, trifluoromethyl, nitro, cyano, alkoxy or amino.

J. Med. Chem. 1996, 39, 4439, J. Med. Chem. 2001, 44, 186, and Bioorg. Med. Chem. Lett. 2003, 13, 1429 relate to computational simulation and pharmacological characterization of some compounds described in WO 96/06846.

The compounds of the invention, described below, are structurally different from the compounds described in WO 96/06846 because of the novel substituents present on the piperazine ring at the 2 position. These structural variations are neither disclosed nor suggested by WO 96/06846, or in Lopez-Rodriguez et al, J. Med. Chem. 1996, 39, 4439, J. Med. Chem. 2001, 44, 186, and Bioorg. Med. Chem. Lett. 2003, 13, 1429. These structural variations result in compounds that are useful as 5-HT_{1A} ligands, with a remarkable affinity for the serotoninergic 5-HT_{1A} receptor and selectivity over α₁ adrenergic receptors.

### SUMMARY OF THE INVENTION

According to the first aspect of the present invention, it provides arylpiperazine derivatives of formula la: 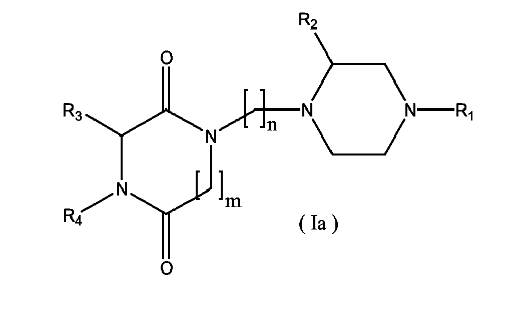 wherein:
m is an integer from 0 to 1;
R₃ and R₄ are H or are methylene groups bound together forming with the heterocyclic ring a 5- or 6- membered ring;
n is an integer from 1 to 4;
R₁ is selected from naphth-1-yl; naphth-2-yl, benzodioxepin-6-yl, benzodioxan-4-yl, benzimidazol-4-yl, dihydro-2*H*-1,5-benzodioxan-5-yl, 7-benzofuranyl, tetrahydronaphthyl or phenyl, wherein phenyl, tetrahydronaphthyl and naphtyl are each optionally substituted with one or more groups chosen from (C₁-C₆)-alkoxy, (C₁-C₆)alkyl, halogen, (C₂-C₆)-alkenyl, halo-(C₁-C₆)-alkyl, phenyl, phenyl(C₁-C₆)-alkyl, phenoxy, (C₁-C₆)-alkylcarbonyl, phenylcarbonyl, phenyl(C₁-C₆)alkylcarbonyl, (C₁-C₆)-alkoxycarbonyl, phenyl(C₁-C₆)alkoxycarbonyl, (C₁-C₆)-alkylcarbonylamino, hydroxy, cyano, nitro, amino, carboxy, sulfo, sulfamoyl, sulfonylamino, (C₁₋C₆)alkylaminosulfonyl or (C₁-C₆)alkylsulfonylamino; and
R₂ is selected from (C₁-C₄)alkyl, (C₂-C₄)alkenyl, (C₁-C₄)alkoxy, halo-(C₁-C₄)alkyl, halogen, hydroxyl, amino, cyano;
their stereoisomers, N-oxides, crystalline forms, hydrates, pharmaceutically acceptable salts and pharmaceutically acceptable solvates.

A second aspect of the invention relates to a pharmaceutical composition comprising an effective amount of a compound of formula la, their stereoisomers, N-oxides, crystalline forms, hydrates, pharmaceutically acceptable salts and pharmaceutically acceptable solvates or mixtures in combination with pharmaceutically acceptable carriers. Accordingly, the present invention relates to a pharmaceutical composition as defined above for the treatment and/or prophylaxis of Parkinson Disease, cerebral damage by thromboembolic ictus, craneoencephalic traumatisms, depression, migraine, pain, psychosis, anxiety disorders, aggressive disorders or urinary tract disorders, particularly urinary incontinence.

A third aspect of the invention relates to use of a compound of formula la, their stereoisomers, N-oxides, crystalline forms, hydrates, pharmaceutically acceptable salts and pharmaceutically acceptable solvates as described herein, for the manufacture of a medicament for the treatment and/or prophylaxis of Parkinson Disease, cerebral damage by thromboembolic ictus, craneoencephalic traumatisms, depression, migraine, pain, psychosis, anxiety disorders, aggressive disorders or urinary tract disorders, particularly urinary incontinence.

This third aspect may alternatively be formulated as a method for treatment of the diseases mentioned above in a human comprising administering to a human in need thereof an effective amount of pharmaceutical product as described herein.

A fourth aspect of the invention relates to processes for the preparation of the compounds of formula la as defined herein which comprises one of the following:
i) reacting a compound of formula II 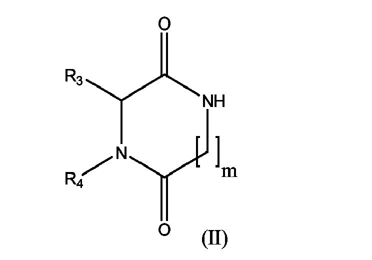 wherein
   m, R₃ and R₄ are as defined above;
   with a compound of formula (IV) 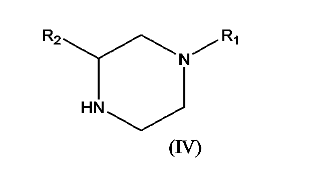 wherein R₁ and R₂ are as defined above;
   resulting in final products of formula la wherein n = 1;
   or
ii) reacting a compound of formula (III) 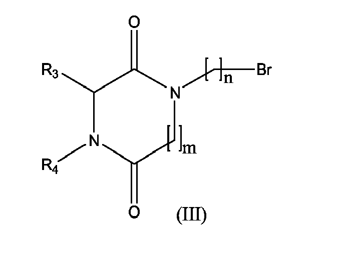 wherein R₃, R₄, and m are as defined above; and n > 1;
   with a compound of formula (IV) as defined above;
   resulting in final compounds of formula la wherein n > 1;
   or
iii) acidifying a basic compound of formula la with a pharmaceutically acceptable acid to give a pharmaceutically acceptable salt;
   or
iv) separating a mixture of isomers of a compound of formula la to isolate one of such isomers substantially free from the other isomer.

Processes i) and ii) are illustrated in Scheme 1, wherein step c) correspond to process i) and steps a) and b) corresponds to process ii).

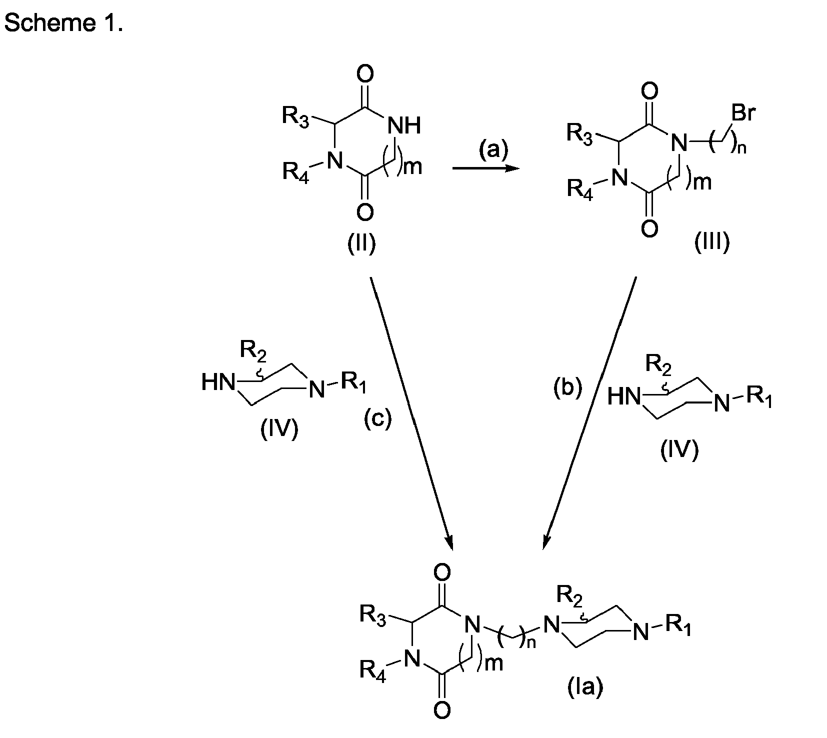

### Scheme 1. Reagents and conditions: (a) Halogen-(CH₂)n-Halogen, NaH, DMF, 110 °C, 1-3 h. (b) NEt₃, CH₃CN, 60 °C, 20 h. (c) HCHO aq, EtOH, reflux, 6 h.

### DETAILED DESCRIPTION OF THE INVENTION:

The inventors have surprisingly identified a class of compounds with a high affinity for the 5-HT_{1A} receptor and remarkable neuroprotective properties.

### Definitions

Prior to a discussion of the detailed embodiments of the invention is provided a definition of specific terms related to the main aspects of the invention.

The term "pharmaceutically acceptable salt", as used herein, refers to salts derived from organic and inorganic acids. The compound of the general formula la may be converted into its pharmaceutically acceptable salts, or its pharmaceutically acceptable solvates by conventional methods. For example, such salts may be prepared by treating one or more of the compounds with an aqueous solution of the desired pharmaceutically acceptable metallic hydroxide or other metallic base and evaporating the resulting solution to dryness, preferably under reduced pressure in a nitrogen atmosphere. Alternatively, a solution of the compound of formula la may be mixed with an alkoxide of the desired metal, and the solution subsequently evaporated to dryness. The pharmaceutically acceptable hydroxides, bases, and alloxides include those worth cations for this purpose, including (but not limited to), potassium, sodium, ammonium, calcium, and magnesium. Other representative pharmaceutically acceptable salts include hydrochloride, hydrobromide, sulphate, bisulphate, lactate, phosphate, tosylate, citrate, maleate, fumarate, succinate, tartrate, acetate, oxalate, propionate, nitrate, methanesulfonate, benzoate and similarly known acceptable acids.

According to the first aspect of the present invention, it provides arylpiperazine derivatives of formula la: 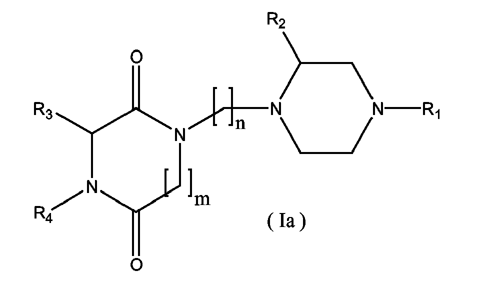 wherein:
m is an integer from 0 to 1;
R₃ and R₄ are H or are methylene groups bound together forming with the heterocyclic ring a 5- or 6- membered ring;
n is an integer from 1 to 4;
R₁ is selected from naphth-1-yl; naphth-2-yl, benzodioxepin-6-yl, benzodioxan-4-yl, benzimidazol-4-yl, dihydro-2*H*-1,5-benzodioxan-5-yl, 7-benzofuranyl, tetrahydronaphthyl or phenyl, wherein phenyl, tetrahydronaphthyl and naphtyl are each optionally substituted with one or more groups chosen from (C₁-C₆)-alkoxy, (C₁-C₆)alkyl, halogen, (C₂-C₆)-alkenyl, halo-(C₁-C₆)-alkyl, phenyl, phenyl(C₁-C₆)-alkyl, phenoxy, (C₁-C₆)-alkylcarbonyl, phenylcarbonyl, phenyl(C₁-C₆)alkylcarbonyl, (C₁-C₆)-alkoxycarbonyl, phenyl(C₁-C₆)alkoxycarbonyl, (C₁-C₆)-alkylcarbonylamino, hydroxy, cyano, nitro, amino, carboxy, sulfo, sulfamoyl, sulfonylamino, (C₁₋C₆)alkylaminosulfonyl or (C₁-C₆)alkylsulfonylamino; and
R₂ is selected from (C₁-C₄)alkyl, (C₂-C₄)alkenyl, (C₁-C₄)alkoxy, halo-(C₁-C₄)alkyl, halogen, hydroxyl, amino, cyano;
their stereoisomers, N-oxides, crystalline forms, hydrates, pharmaceutically acceptable salts and pharmaceutically acceptable solvates.

According to an embodiment of the first aspect of the invention, it relates to arylpiperazine derivatives according to formula la, as defined above, wherein R₃ and R₄ are methylene groups bound together forming with the heterocyclic ring a 5- membered ring.

According to another embodiment of the first aspect of the invention, it relates to arylpiperazine derivatives according to formula la, as defined above, wherein m = 1.

According to another embodiment of the first aspect of the invention, it relates to arylpiperazine derivatives according to formula la, as defined above, wherein R₃ and R₄ are methylene groups bound together forming with the heterocyclic ring a 5- membered ring; and m = 1.

In a preferred embodiment, R₁ is selected from unsubstituted naphth-1-yl, benzimidazol-4-yl and benzodioxepin-6-yl.

In a particularly preferred embodiment of the invention, R₁ is unsubstituted naphth-1-yl.

In a more preferred embodiment, R₂ = (C₁-C₄)alkyl.

The term "(C₁-C₄)alkyl" as used herein refers to a saturated branched or linear hydrocarbon chain with 1 to 4 hydrocarbon atoms. Preferably "(C₁₋C₄)alkyl" is an unsubstituted group selected from methyl, ethyl, propyl, isopropyl, butyl, isobutyl, *s*-butyl and *t*-butyl.

According to another embodiment of the invention, n is 1 or 4.

Accordingly, in a preferred embodiment of the present invention, R₃ and R₄ are methylene groups bound together forming with the heterocyclic ring a 5- membered ring; m = 1; R₁ is selected from unsubstituted naphth-1-yl and benzodioxepin-6-yl; R₂ = (C₁-C₄)alkyl; their stereoisomers, N-oxides, crystalline forms, hydrates, pharmaceutically acceptable salts and pharmaceutically acceptable solvates.

Accordingly, in a more specific embodiment of the present invention, it relates to compounds of formula la wherein R₃ and R₄ are methylene groups bound together forming with the heterocyclic ring a 5- membered ring; m = 1; n = 1; R₁ is unsubstituted naphth-1-yl; R₂ = (C₁-C₄)alkyl; their stereoisomers, N-oxides, crystalline forms, hydrates, pharmaceutically acceptable salts and pharmaceutically acceptable solvates.

Furthermore, compounds wherein R₃ and R₄ are methylene groups bound together forming with the heterocyclic ring a 5- membered ring; m = 1; n = 4; R₁ is naphth-1-yl; R₂ = (C₁-C₄)alkyl; their stereoisomers, N-oxides, crystalline forms, hydrates, pharmaceutically acceptable salts and pharmaceutically acceptable solvates, are particularly preferred.

The following compounds are particularly preferred:
**(a)** (2*R*,8a*RS*)-2-[4-[4-(Naphth-1-yl)-2-methylpiperazin-1-yl]butyl]-1,4-dioxoperhydropyrrolo[1,2-a]pyrazine;
**(b)** (2*S*,8a*RS*)-2-[4-[4-(Naphth-1-yl)-2-methylpiperazin-1-yl]butyl]-1,4-dioxoperhydropyrrolo[1,2-a]pyrazine;
**(c)** (2*R*,8a*R*)-2-[[4-(Naphth-1-yl)-2-methylpiperazin-1-yl]methyl]-1,4-dioxoperhydropyrrolo[1,2-a]pyrazine;
**(d)** (2*S*,8a*S*)-2-[[4-(Naphth-1-yl)-2-methylpiperazin-1-yl]methyl]-1,4-dioxoperhydropyrrolo[1,2-a]pyrazine;
**(e)** (2R,8aS)-2-[[4-(Naphth-1-yl)-2-methylpiperazin-1-yl]methyl]-1,4-dioxoperhydropyrrolo[1,2-a]pyrazine;
**(f)** (2*S*,8a*R*)-2-[[4-(Naphth-1-yl)-2-methylpiperazin-1-yl]methyl]-1,4-dioxoperhydropyrrolo[1,2-a]pyrazine.

Also, the inventors have discovered some compounds with an unsubstituted piperazine ring but which, compared to the prior art compounds disclosed in the references cited above, have particularly promising pharmacological properties. These compounds do thus also form a part of the invention:
**(g)** 2-[4-[4-(Naphth-1-yl)piperazin-1-yl]butyl]-1,4-dioxoperhydropyrrolo[1,2-a]pyrazine: 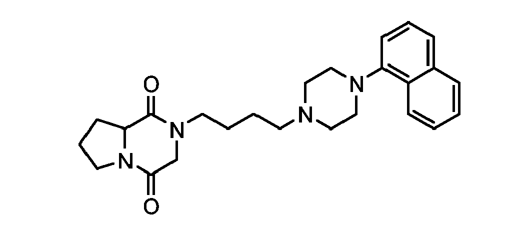
Compound (g) binds in the picomolar range to the 5-HT_{1A} receptor and in the low nanomolar range to the α₁ adrenoceptor (see table 1), which makes the compound be a particularly interesting compound for the treatment of e.g. urinary incontinence.
**(h)** 2-[4-[4-(3,4-Dihydro-2*H*-1,5-benzodioxepin-6-yl)piperazin-1-yl]butyl]-1,4-dioxoperhydropyrrolo[1,2-*a*]pyrazine 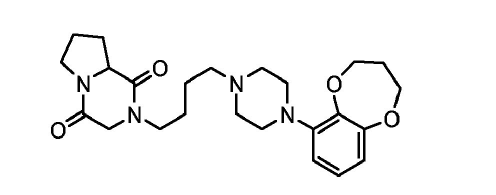
Compound (h) also binds to the 5-HT_{1A} receptor in the low nanomolar range and also binds in the nanomolar range to the α₁ adrenoceptor. This is surprising because the benzodioxepine derivatives in prior art (Bioorg. Med. Chem. Lett. 2003, 13, 1429) do not show substantial binding to the α₁ receptor.

The final products have been structurally characterized by IR, NMR and quantitative elemental analysis techniques. For greater ease of handling, when the final product is not crystalline, it is transformed in a pharmaceutically acceptable salt, derived from an inorganic or organic acid.

It is understood that compounds according to formula la can include asymmetric carbons, and formula la encompasses all possible stereoisomers and mixtures thereof, as well as racemic modifications, particularly those that possess the activity discussed below. Optical isomers may be obtained in pure form by standard separation techniques.

### Pharmaceutical Product

In other embodiments, the invention provides pharmaceutical compositions containing one or more of the compounds of formula la, their stereoisomers, pharmaceutically acceptable salts or pharmaceutically acceptable solvates, and optionally one or more pharmaceutically acceptable carriers, excipients or diluents. The term "carrier", as used herein, shall encompass carriers, excipients and diluents.

Examples of such carriers are well known to those skilled in the art and are prepared accordance with acceptable pharmaceutical procedures. Pharmaceutically acceptable carriers are those carriers that are compatible with the other ingredients in the formulation and are biologically acceptable.

A pharmaceutical product as described herein can be administered orally, transdermally, parenterally, intramuscularly, intravenously, subcutaneously or by other modes of administration. Preferably, the pharmaceutical product can be administered orally.

Representative solid carriers include one or more substance that can act as flavouring agents, lubricants, solubilizers, suspending agents, fillers, glidants, compression aids, binders, tablet-disintegrating agents, or encapsulating materials. Oral formulations containing the active compounds of this invention may comprise any conventionally used oral forms, including tablets, capsules, buccal forms, troches, lozenges and oral liquids, suspensions or solutions. In powders, the carrier is a finely divided solid that is in admixture with the finely divided active ingredient. In tablets, the active ingredient is mixed with a carrier having the necessary compression properties in suitable proportion and compacted in the shape and size desired.

Capsules may contain mixtures of the active compound(s) with inert fillers and/or diluents such as the pharmaceutically acceptable starches, sugars, artificial sweetening agents, powdered celluloses, such as crystalline and microcrystalline celluloses, flours, gelatins, gums, etc.

Useful tablet formulations may be made by conventional compression, wet granulation or dry granulation methods and utilize pharmaceutically acceptable diluents, binding agents, lubricants, disintegrants, surface modifying agents (including surfactants), suspending or stabilizing agents, including, but not limited to, magnesium stearate, stearic acid, sodium lauryl sulfate, microcrystalline cellulose, methyl cellulose, sodium carboxymethyl cellulose, carboxymethylcellulose calcium, polyvinylpyrrolidine, gelatin, alginic acid, acacia gum, xanthan gum, sodium citrate, complex silicates, calcium carbonate, glycine, dextrin, sucrose, sorbitol, dicalcium phosphate, calcium sulfate, lactose, kaolin, mannitol, sodium chloride, talc, starches, sugars, low melting waxes, and ion exchange resins. Preferred surface modifying agents include nonionic and anionic surface modifying agents. Representative examples of surface modifying agents include, but are not limited to, poloxamer 188, benzalkonium. chloride, calcium stearate, cetostearl alcohol, cetomacrogol emulsifying wax, sorbitan esters, colloidol silicon dioxide, phosphates, sodium dodecylsulfate, magnesium aluminium silicate, and triethanolarnine. Oral formulations herein may utilize standard delay or time release formulations to alter the absorption of the active compound(s). The oral formulation may also consist of administering the active ingredient in water or a fruit juice, containing appropriate solubilizers or emulsifiers as needed.

Liquid carriers can be used in preparing solutions, suspensions, emulsions, syrups, and elixirs. The active ingredient can be dissolved or suspended in a pharmaceutically acceptable oil or fat. The liquid carrier can obtain other suitable pharmaceutical additives such as, for example, solubilizers, emulsifiers, buffers, preservatives, sweeteners, flavouring agents, suspending agents, thickening agents, colours, viscosity regulators, stabilizers or osmoregulators. Suitable examples of liquid carriers for oral and parenteral administration include water (particularly containing additives as above, e.g. cellulose derivatives, preferably sodium carboxymethyl cellulose solution), alcohols (including monohydric alcohols and polyhydric alcohols, e.g. glycols) and their derivatives, and oils (e.g. fractionated coconut oil and arachis oil).

For parenteral administration, the carrier can also be an oily ester such as ethyl oleate and isopropyl myristrate. Sterile liquid carriers are used in sterile liquid form compositions for parenteral administration. The liquid carrier for pressurized compositions can be halogenated hydrocarbon or other pharmaceutically acceptable propellant.

The compounds of this invention may also be administered parenterally or intraperitoneally. Solutions or suspensions of these active compounds as a free base or pharmacologically acceptable salt can be prepared in water suitably mixed with a surfactant such as hydroxypropylcellulose. Dispersions can also be prepared in glycerol, liquid polyethylene glycols and mixtures thereof in oils. Under ordinary conditions of storage and use, these preparations contain a preservative to inhibit the growth of microorganisms.

The pharmaceutical forms suitable for injectable use include sterile aqueous solutions or dispersions and sterile powders for the extemporaneous preparation of sterile injectable solutions or dispersions. It must be stable under the conditions of manufacture and storage and must be preserved against the contaminating action of microorganisms such as bacteria and fungi. The carrier can be a solvent or dispersion medium containing, for example, water, ethanol, polyol (e.g., glycerol, propylene glycol and liquid polyethylene glycol), suitable mixtures thereof, and vegetable oils.

The carriers described above are not meant to be exclusive, but instead merely representative of the classes of carriers and the particular carriers that may be used in preferred dosage forms of the present invention.

A pharmaceutical product, as described herein, may include other pharmaceutically active substances. It can be prepared by mixing the active compounds with one or more pharmacologically tolerated carriers and converting the mixture into a suitable pharmaceutical form.

### Use in clinical symptoms

Taking into account its 5-HT_{1A} receptor affinity and its neuroprotective capacity, the compounds of formula la are useful in the treatment and/or prophylaxis of pathological states wherein the 5-HT_{1A} receptor agonists are indicated, such as, for example, the treatment and/or prophylaxis of cerebral damage caused by thromboembolic stroke or traumatic brain damage, as well as the treatment and/or prevention of Parkinson's disease, depression, migraine, pain, psychosis such as e.g. schizophrenia; mood disorders, such as anxiety disorders (e.g. obsessive compulsive disorders, generalized anxiety) and aggressive disorders (including mixed aggressive-anxiety/depressive disorders); and urinary tract disorders, particularly urinary incontinence, in mammals, particularly in humans.

Additional objects, advantages and features of the invention will become apparent to those skilled in the art upon examination of the description or may be learned by practice of the invention. The following examples and drawings are provided by way of illustration, and is not intended to be limiting of the present invention.

### EXAMPLES

### Example 1. Synthesis of compounds of general structure la (n > 1). General procedure. (See scheme 1)

To a suspension of the bromoalkyl derivative **III** (4.5 mmol) and the appropriate arylpiperazine **IV** (7.5 mmol) in dry acetonitrile (10 mL) was added triethylamine (1.0 mL, 7.5 mmol), and the mixture was refluxed for 20-24 h. After cooling down, the solvent was evaporated under reduced pressure and the residue was resuspended in water and extracted with dichloromethane (3 x 50 mL). The combined organic layers were washed with water and dried over anhydrous Na₂SO₄. After evaporation of the solvent the crude oil was purified by column chromatography in silica gel using the appropriate eluent. Collected data of IR and NMR spectra refer to free bases, then hydrochloride salts were prepared prior to mp, elemental analyses and biological assays.

### Example 2. 2-[4-[4-(Naphth-1-yl)piperazin-1-yl]butyl]-1,4-dioxoperhydropyrrolo[1,2-a]pyrazine (g).

The title compound was prepared following general procedure described in example 1, starting from 2-(4-bromobutyl)-1,4-dioxoperhydropyrrolo[1,2-a]pyrazine and 1-(naphth-1-yl)piperazine as reagents,
Chromatography: chloroform/methanol, from 9.5:0.5 to 9:1.
Yield: 43%; mp: 277-280 °C (d) (methanol/ethyl ether).
IR (CHCl₃): 1670, 1600, 1580, 1460 cm⁻¹.
¹H NMR (CDCl₃): δ 1.56-1.65 (m, 4H), 1.88-2.12 (m, 3H), 2.35-2.41 (m, 1 H), 2.51 (t, J = 6.9, 2H), 2.74 (br s, 4H), 3.15 (br s, 4H), 3.33-3.41 (m, 1 H), 3.50-3.63 (m, 3H), 3.79 (d, J = 16.2, 1 H), 4.07 (t, J = 7.8, 1 H), 4.14 (d, J = 16.2, 1 H), 7.09 (dd, J = 7.3, 1.0, 1 H), 7.39 (t, J = 7.8, 1 H), 7.44-7.48 (m, 2H), 7.55 (d, J = 8.4, 1 H), 7.80-7.83 (m, 1 H), 8.17-8.20 (m, 1 H).
¹³C NMR (CDCl₃): δ 22.6, 23.8, 25.0, 28.8, 45.2, 45.9, 51.6, 52.7, 53.6, 58.0, 59.0, 114.6, 123.4 (2 C), 125.2, 125.7, 125.8, 128.3, 128.7, 134.6, 149.4, 163.1, 167.1.
Anal. calculated for C₂₅H₃₂N₄O₂·HCl·1/2H₂O: C, 64.43, H, 7.35, N, 12.02; found: C, 64.57, H, 7.20, N, 11.89.

### Example 3. (2R,8aRS)-2-[4-[4-(Naphth-1-yl)-2-methylpiperazin-1-yl]butyl]-1,4-dioxoperhydropyrrolo[1,2-a]pyrazine, (a) (mixture of diastereoisomers).

The title compound was prepared following general procedure described in example 1, starting from 2-(4-bromobutyl)-1,4-dioxoperhydropyrrolo[1,2-a]pyrazine and (R)-3-methyl-1-(naphth-1-yl)piperazine as reagents,
Chromatography: chloroform/ethanol, from 20:1 to 12:1.
Yield: 57% (oil); [α]_{D}²⁵ = -20.0 (c = 1.1, CHCl₃).
IR (CHCl₃): 1665, 1575, 1510, 1460 cm⁻¹.
¹H NMR (CDCl₃): δ 1.16 (d, J = 5.6, 3H), 1.50-1.60 (m, 4H), 1.80-2.14 (m, 3H), 2.30-2.45 (m, 2H), 2.59-2.89 (m, 4H), 3.03-3.08 (m, 2H), 3.18-3.23 (m, 2H), 3.28-3.67 (m, 4H), 3.77 (dd, J = 16.4, 2.2, 1 H), 4.03 (t, J = 7.6, 1 H), 4.14 (d, J = 16.4, 1 H), 7.05 (dd, J = 7.6, 1.2, 1 H), 7.36 (t, J = 8.1, 1 H), 7.38-7.46 (m, 2H), 7.51 (d, J = 8.4, 1 H), 7.74-7.81 (m, 1 H), 8.13-8.18 (m, 1 H).
¹³C NMR (CDCl₃): δ 22.6 (2 C), 23.1, 25.1, 28.8, 29.6, 45.2, 45.9 (2 C), 51.6 (2 C), 53.0, 55.4, 59.0, 60.1, 114.6, 123.4 (2 C), 125.2, 125.7, 125.8, 128.3, 128.8, 134.6, 149,3, 163.1, 167.1.

### Example 4. (2S,8aRS)-2-[4-[4-(Naphth-1-yl)-2-methylpiperazin-1-yl]butyl]-1,4-dioxoperhydropyrrolo[1,2-a]pyrazine, (b) (mixture of diastereoisomers).

The title compound was prepared following general procedure described in example 1, starting from 2-(4-bromobutyl)-1,4-dioxoperhydropyrrolo[1,2-a]pyrazine and (*S*)-3-methyl-1-(naphth-1-yl)piperazine as reagents,
Chromatography: chloroform/ethanol, from 20:1 to 12:1.
Yield: 35% (oil). [α]_{D}²⁵ +21.0 (c = 1.1, CHCl₃).
Spectral data are identical to those of 3 (see above).

### Example 5. 2-[4-[4-(3,4-Dihydro-2H-1,5-benzodioxepin-6-yl)piperazin-1-yl]butyl]-1,4-dioxoperhydropyrrolo[1,2-a]pyrazine, (h).

The title compound was prepared following general procedure described in example 1, starting from 2-(4-bromobutyl)-1,4-dioxoperhydropyrrolo[1,2-a]pyrazine and 1-(3,4-dihydro-2*H*-1,5-benzodioxepin-6-yl)piperazine as reagents,
Chromatography: chloroform/methanol, from 9.5:0.5 to 9:1.
Yield 52%. mp 212-213 °C (d) (methanol/ethyl ether).
IR (CHCl₃): 1670, 1590, 1485, 1460 cm⁻¹.
¹H NMR (CDCl₃): δ 1.43-1.55 (m, 4H), 1.82-2.05 (m, 3H), 2.13 (qt, J = 5.7, 2H), 2.27-2.31 (m, 1 H), 2.35 (t, J = 7.2, 2H), 2.55 (br s, 4H), 3.00 (br s, 4H), 3.27-3.34 (m, 1 H), 3.41-3.57 (m, 3H), 3.72 (d, J = 16.2, 1 H), 4.01 (t, J = 7.5, 1 H), 4.07 (d, J = 16.5, 1 H), 4.14-4.21 (m, 4H), 6.54 (dd, J = 7.8, 1.5, 1 H), 6.59 (dd, J = 8.2, 1.4, 1 H), 6.76 (t, J = 7.9, 1 H).
¹³C NMR (CDCl₃): δ 22.6, 23.9, 25.1, 28.8, 31.5, 45.2, 46.0, 51.0, 51.6, 53.4, 58.0, 59.0, 70.2, 70.3, 112.9, 115.5, 122.5, 144.6, 145.0, 152.1, 163.1, 167.1.
Anal. calculated for C₂₄H₃₄N₄O₄.2HCl.2H₂O: C, 52.26, H, 7.31, N, 10.15; found: C, 52.02, H, 6.93, N, 10.07.

### Example 6. Synthesis of compounds of general structure la (n = 1). General procedure. (See scheme 1)

To a suspension of intermediate **II** (7 mmol) and formaldehyde (7 mmol from a 35% aqueous solution) in methanol (15 mL) was added the corresponding arylpiperazine **IV** (7 mmol). The resultant suspension was refluxed for 2-6 hours after complete disappearance of the starting materials (TLC). The mixture was then cooled to room temperature, and the solvent was evaporated at reduced pressure. The crude mixture was diluted in chloroform (75 mL) and washed with water (3 x 75 mL). The organic layer was dried over anhydrous Na₂SO₄, filtered and evaporated at reduced pressure. The obtained crude was purified by column chromatography on silica gel using the appropriate eluent. Collected data of IR and NMR spectra refer to free bases, then hydrochloride salts were prepared prior to mp, elemental analyses and biological assays.

### Example 7. (2R,8aR)-2-[[4-(Naphth-1-yl)-2-methylpiperazin-1-yl]methyl]-1,4-dioxoperhydropyrrolo[1,2-a]pyrazine, (c)

The title compound was prepared following general procedure described in example 6, starting from (R)-1,4-dioxoperhydropyrrolo[1,2-a]pyrazine and (R)-3-methyl-1-(naphth-1-yl)piperazine as reagents,
Chromatography: from ethyl acetate to ethyl acetate/ethanol, 9:1.
Yield: 23%. [α]_{D}²⁵ =+36.6 (c = 1.9, CHCl₃).
IR (CHCl₃): 1665, 1595, 1575, 1500, 1455 cm⁻¹.
¹H NMR (CDCl₃): δ 1.22 (d, J = 7.2, 3H), 1.80-1.94 (m, 1 H), 1.96-2.16 (m, 2H), 2.32-2.42 (m, 2H), 2.72-2.79 (m, 2H), 2.88-3.02 (m, 2H), 3.14-3.28 (m, 2H), 3.49-3.69 (m, 2H), 4.00-4.15 (m, 3H), 4.25 (d, J = 12.3, 1 H), 4.40 (d, *J* = 12.3, 1 H), 7.02 (dd, J = 7.5, 0.9, 1 H), 7.37 (t, J = 7.5, 1 H), 7.42-7.46 (m, 2H), 7.52 (d, J = 8.1, 1 H), 7.78-7.81 (m, 1 H), 8.15-8.19 (m, 1 H).
¹³C NMR (CDCl₃): δ 22.7 (2 C), 28.6, 45.1, 45.2, 51.0, 52.9, 53.5, 59.1, 60.3, 63.7, 114.6, 123.3, 123.5, 125.3, 125.7 (2 C), 128.3, 128.7, 134.6, 149.2, 163.8, 168.1.
Anal. calculated for C₂₃H₂₈N₄O₂.HCl.5/2H₂O: C, 58.27, H, 7.23, N, 11.82; found: C, 58.25, H, 6.80, N, 11.50.

### Example 8. (2S,8aS)-2-[[4-(Naphth-1-yl)-2-methylpiperazin-1-yl]methyl]-1,4-dioxoperhydropyrrolo[1,2-a]pyrazine, (d)

The title compound was prepared following general procedure described in example 6, starting from (*S*)-1,4-dioxoperhydropyrrolo[1,2-a]pyrazine and (*S*)-3-methyl-1-(naphth-1-yl)piperazine as reagents,
Yield: 36%. [α]_{D}²⁵ =-38.0 (*c* = 1.2, CHCl₃).
Spectral data are identical to those of (c) (see above).
Anal. calculated for C₂₃H₂₈N₄O₂.HCl.2/3H₂O: C, 62.64, H, 6.88, N, 12.71; found: C, 62.51, H, 7.04, N, 12.97.

### Example 9. (2R,8aS)-2-[[4-(Naphth-1-yl)-2-methylpiperazin-1-yl]methyl]-1,4-dioxoperhydropyrrolo[1,2-a]pyrazine, (e)

The title compound was prepared following general procedure described in example 6, starting from (S)-1,4-dioxoperhydropyrrolo[1,2-a]pyrazine and (*R*)-3-methyl-1-(naphth-1-yl)piperazine as reagents,
Chromatography: from ethyl acetate to ethyl acetate/ethanol, 9:1.
Yield: 43%. [α]_{D}²⁵ =-53.2 (c = 1.4, CHCl₃).
IR (CHCl₃): 1665, 1595, 1575, 1500, 1455 cm⁻¹.
¹H NMR (CDCl₃): δ 1.22 (m, 3H), 1.85-1.94 (m, 1 H), 1.96-2.17 (m, 2H), 2.34-2.42 (m, 2H), 2.62-2.80 (m, 2H), 2.85-3.04 (m, 2H); 3.14-3.24 (m, 2H), 3.51-3.68 (m, 2H), 4.05-4.18 (m, 3H), 4.28-4.38 (m, 2H), 7.03 (d, *J* = 7.2, 1 H), 7.37 (t, J = 7.8, 1 H), 7.43-7.46 (m, 2H), 7.52 (d, J = 8.1, 1 H), 7.78-7.81 (m, 1 H), 8.15-8.18 (m, 1H).
¹³C NMR (CDCl₃): δ 22.6 (2 C), 28.7, 45.1 (2 C), 50.6, 53.0, 53.4, 59.1, 60.3, 63.2, 114.6, 123.3, 123.5, 125.3, 125.8 (2 C), 128.3, 128.7, 134.6, 149.2, 163.6, 168.0.
Anal. calculated for C₂₃H₂₈N₄O₂.HCl.H₂O: C, 61.80, H, 6.99, N, 12.54; found: C, 61.47, H, 7.06, N, 12.54.

### Example 10. (2S,8aR)-2-[[4-(Naphth-1-yl)-2-methylpiperazin-1-yl]methyl]-1,4-dioxoperhydropyrrolo[1,2-a]pyrazine, (f)

The title compound was prepared following general procedure described in example 6, starting from (*R*)-1,4-dioxoperhydropyrrolo[1,2-*a*]pyrazine and (*S*)-3-methyl-1-(naphth-1-yl)piperazine as reagents,
Yield: 40%. [α]_{D}²⁵ =+53.7 (*c* = 0.9, CHCl₃).
Spectral data are identical to those of (e) (see above).
Anal. calculated for C₂₃H₂₈N₄O₂.HCl.3/2H₂O: C, 60.58, H, 7.07, N, 12.29; found: C, 60.72, H, 7.13, N, 12.04.

### Example 11. Radioligand Binding Assays.

For all receptor binding assays, male Sprague-Dawley rats (*Rattus norvegicus albinus*), weighing 180-200 g, were killed by decapitation and the brains rapidly removed and dissected. Tissues were stored at -80 °C for subsequent use and homogenized on a Polytron PT-10 homogenizer. Membrane suspensions were centrifuged on a Beckman J2-HS instrument.

### 5-HT_{1A} Receptor.

The cerebral cortex was homogenized in 10 volumes of ice-cold Tris buffer (50 mM Tris-HCl, pH 7.7 at 25 °C) and centrifuged at 28000g for 15 min. The membrane pellet was washed twice by resuspension and centrifugation. After the second wash the resuspended pellet was incubated at 37 °C for 10 min. Membranes were then collected by centrifugation and the final pellet was resuspended in 50 mM Tris-HCl, 5 mM MgSO₄, and 0.5 mM EDTA buffer (pH 7.4 at 37 °C). Fractions of 100 µL of the final membrane suspension (about 1 mg of protein) were incubated at 37 °C for 15 min with 0.6 nM [³H]-8-OH-DPAT (133 Ci/mmol), in the presence or absence of the competing drug, in a final volume of 1.1 mL of assay buffer (50 mM Tris-HCl, 10 nM clonidine, 30 nM prazosin, pH 7.4 at 37 °C). Nonspecific binding was determined with 10 µM 5-HT.

### α₁ Adrenoceptor.

The cerebral cortex was homogenized in 20 volumes of ice-cold buffer (50 mM Tris-HCl, 10 mM MgCl₂, pH 7.4 at 25 °C) and centrifuged at 30000*g* for 15 min. Pellets were washed twice by resuspension and centrifugation. Final pellets were resuspended in the same buffer. Fractions of the final membrane suspension (about 250 µg of protein) were incubated at 25 °C for 30 min with 0.2 nM [³H]prazosin (23 Ci/mmol), in the presence or absence of six concentrations of the competing drug, in a final volume of 2 mL of buffer. Nonspecific binding was determined with 10 µM phentolamine.

For all binding assays, competing drug, nonspecific, total and radioligand bindings were defined in triplicate. Incubation was terminated by rapid vacuum filtration through Whatman GF/B filters, presoaked in 0.05% poly(ethylenimine), using a Brandel cell harvester. The filters were then washed with the assay buffer, dried and placed in poly(ethylene) vials to which were added 4 mL of a scintillation cocktail (Aquasol). The radioactivity bound to the filters was measured by liquid scintillation spectrometry. The data were analyzed by an iterative curve-fitting procedure (program Prism, Graph Pad), which provided IC₅₀, *K*ᵢ, and *r*² values for test compounds, *K*ᵢ values being calculated from the Cheng and Prusoff equation. The protein concentrations of the rat cerebral cortex and the rat striatum were determined by the method of Lowry, using bovine serum albumin as the standard.

Results from these assays are presented below in Table 1.

**Table 1. Binding data of compounds la**

| Compound | *K*ᵢ± SEM (5-HT_{1A}) | *K*ᵢ± SEM (α₁) |
|---|---|---|
| **(g)** | 0.5 ± 0.2 | 8.0 ± 1.7 |
| **(a)** | 4.2 ± 0.9 | 20.1 ± 0.8 |
| **(b)** | 15.3 ±1.8 | 34.4 ±1.2 |
| **(h)** | 3.1 ±0.9 | 348 ± 21 |
| **(c)** | 9.5±2.2 | >1000 |
| **(d)** | 2.5 ±0.1 | >1000 |
| **(e)** | 6.3±0.2 | >1000 |
| **(f)** | 6.4 ±0.1 | >1000 |

| | | |
|---|---|---|
| Values are means of 2-4 experiments performed in triplicate. | | |

## Claims

1. A compound of formula la: 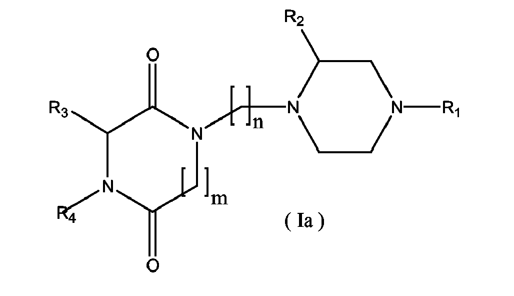 wherein:
m is an integer from 0 to 1;
R₃ and R₄ are H or are methylene groups bound together forming with the heterocyclic ring a 5- or 6- membered ring;
n is an integer from 1 to 4;
R₁ is selected from naphth-1-yl; naphth-2-yl; benzodioxepin-6-yl,
benzodioxan-4-yl, benzimidazol-4-yl, dihydro-2*H*-1,5-benzodioxan-5-yl, 7-benzofuranyl, tetrahydronaphthyl or phenyl, wherein phenyl, tetrahydronaphthyl and naphtyl are each optionally substituted with one or more groups chosen from (C₁-C₆)-alkoxy, (C₁-C₆)alkyl, halogen, (C₂-C₆)-alkenyl, halo-(C₁-C₆)-alkyl, phenyl, phenyl(C₁-C₆)-alkyl, phenoxy, (C₁-C₆)-alkylcarbonyl, phenylcarbonyl, phenyl(C₁-C₆)alkylcarbonyl, (C₁-C₆)-alkoxycarbonyl, phenyl(C₁-C₆)alkoxycarbonyl, (C₁-C₆)-alkylcarbonylamino, hydroxy, cyano, nitro, amino, carboxy, sulfo, sulfamoyl, sulfonylamino, (C₁₋C₆)alkylaminosulfonyl or (C₁-C₆)alkylsulfonylamino; and
R₂ is selected from (C₁-C₄)alkyl, (C₂-C₄)alkenyl, (C₁-C₄)alkoxy,
halo-(C₁-C₄)alkyl, halogen, hydroxyl, amino, cyano;
their stereoisomers, N-oxides, crystalline forms, hydrates, pharmaceutically acceptable salts and pharmaceutically acceptable solvates.

2. The compound according to claim 1, wherein R₃ and R₄ are methylene groups bound together forming with the heterocyclic ring a 5- membered ring.

3. The compound according to any of the claims 1 or 2, wherein m = 1.

4. The compound according to any of claims 1 to 2, wherein R₁ is selected from unsubstituted naphth-1-yl, benzimidazol-4-yl and benzodioxepin-6-yl.

5. The compound according to any of claims 1 to 3, wherein R₂ = (C₁-C₄)alkyl.

6. The compound according to any of claims 1 to 5, wherein:
R₃ and R₄ are methylene groups bound together forming with the heterocyclic ring a 5- membered ring;
m = 1;
n=1;
R₁ is naphth-1-yl;
R₂ = (C₁-C₄)alkyl.

7. The compound according to any of claims 1 to 5, wherein:
R₃ and R₄ are methylene groups bound together forming with the heterocyclic ring a 5- membered ring;
m = 1;
n = 4;
R₁ is naphth-1-yl;
R₂ = (C₁-C₄)alkyl.

8. The compound according to claim 1, which is selected from the following:
**(a)** (2*R*,8a*RS*)-2-[4-[4-(Naphth-1-yl)-2-methylpiperazin-1-yl]butyl]-1,4-dioxoperhydropyrrolo[1,2-a]pyrazine;
**(b)** (2*S*,8a*RS*)-2-[4-[4-(Naphth-1-yl)-2-methylpiperazin-1-yl]butyl]-1,4-dioxoperhydropyrrolo[1,2-a]pyrazine;
**(c)** (2*R*,8a*R*)-2-[[4-(Naphth-1-yl)-2-methylpiperazin-1-yl]methyl]-1,4-dioxoperhydropyrrolo[1,2-a]pyrazine;
**(d)** (2*S*,8a*S*)-2-[[4-(Naphth-1-yl)-2-methylpiperazin-1-yl]methyl]-1,4-dioxoperhydropyrrolo[1,2-*a*]pyrazine;
**(e)** (2*R*,8a*S*)-2-[[4-(Naphth-1-yl)-2-methylpiperazin-1-yl]methyl]-1,4-dioxoperhydropyrrolo[1,2-*a*]pyrazine;
**(f)** (2*S*,8a*R*)-2-[[4-(Naphth-1-yl)-2-methylpiperazin-1-yl]methyl]-1,4-dioxoperhydropyrrolo[1,2-*a*]pyrazine.

9. The compound according to claim 1, which is selected from the following:
**(g)** 2-[4-[4-(Naphth-1-yl)piperazin-1-yl]butyl]-1,4-dioxoperhydropyrrolo[1,2-a]pyrazine;
**(h)** 2-[4-[4-(3,4-Dihydro-2*H*-1,5-benzodioxepin-6-yl)piperazin-1-yl]butyl]-1,4-dioxoperhydropyrrolo[1,2-*a*]pyrazine.

10. A pharmaceutical composition comprising an effective amount of a compound of formula la, their stereoisomers, N-oxides, crystalline forms, hydrates, pharmaceutically acceptable salts, pharmaceutically acceptable solvates or mixtures according to any of claims 1 to 9, in combination with one or more pharmaceutically acceptable carriers.

11. Use of a compound according to any of claims 1 to 9, for the manufacture of a medicament for the treatment and prophylaxis of a very wide range of disorders mediated by 5-HT_{1A} receptors and associated clinical symptoms in a human person.

12. The use according to claim 10, wherein such disorders are selected from Parkinson Disease, cerebral damage by thromboembolic ictus, craneoencephalic traumatisms, depression, migraine, pain, psychosis, anxiety disorders, aggressive disorders or urinary tract disorders.

13. A process for the preparation of compounds of formula la according to any of claims 1 to 9, which comprises one of the following:
i) reacting a compound of formula II 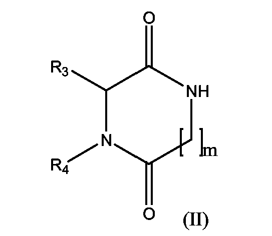 wherein m, R₃ and R₄are as defined above ;
with a compound of formula (IV) 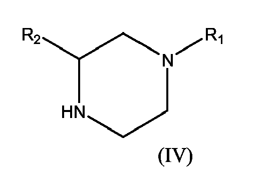 wherein R₁ and R₂ are as defined above;
resulting in final products of formula la wherein n = 1;
or
ii) reacting a compound of formula (III) 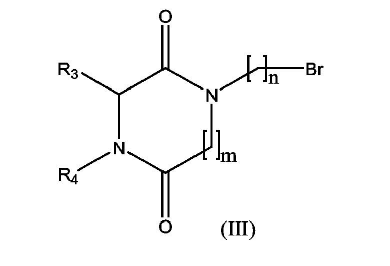 wherein R₃, R₄ and m are as defined above; and n > 1;
with a compound of formula (IV) as defined above;
resulting in final products of formula la wherein n > 1;
or
iii) acidifying a basic compound of formula la with a pharmaceutically acceptable acid to give a pharmaceutically acceptable salt;
or
iv) separating a mixture of isomers of a compound of formula la to isolate one of such isomers substantially free from the other isomer.
